# EUROPEAN PATENT APPLICATION

(11) **EP 4 134 673 A1**
(43) Date of publication of application: **15.02.2023**
(21) Application number: 21784770.6
(22) Date of filing: 12.04.2021
(51) Int. Cl.: G01N 33/68, G01N 33/53

(54) **COMPOSITION USING URINE SAMPLE FOR DIAGNOSIS OF KIDNEY DISEASE**

(30) Priority: 10.04.2020 KR 20200043706
(71) Applicant: Bioneer Corporation, Daejeon 34302 (KR); Sirnagen Therapeutics Corporation, Daejeon 34302 (KR); Soonchunhyang University Industry Academy Cooperation Foundation, Asan-si, Chungcheongnam-do 31358 (KR)
(72) Inventor: SON, Seung Seob, Cheonan-si Chungcheongnam-do 31156 (KR); KIM, Tae Rim, Daejeon 35217 (KR); YUN, Sung Il, Daejeon 34022 (KR); PARK, Jun Hong, Daejeon 34538 (KR); PARK, Han-Oh, Sejong 30151 (KR); CHO, Nam-Jun, Cheonan-si Chungcheongnam-do 31156 (KR); LEE, Eun Young, Seoul 06501 (KR)
(74) Representative: Mewburn Ellis LLP
(86) International application number: PCT/KR2021/004557
(87) International publication number: WO 2021/206523

(57) **Abstract**

The present invention relates to a composition using a urine sample for diagnosis of a kidney disease and, more specifically, to a urine test composition and a kit for diagnosis of a kidney disease, each comprising an agent specifically detecting amphiregulin from a urine sample, and a method for diagnosing a kidney disease by using the composition. According to the present invention, not only is there an advantage of being able to conveniently diagnose a kidney disease in a non-invasive manner using a urine sample for a kidney disease to which early diagnosis is important, but also the application of the present invention to a patient diagnosed with chronic kidney disease can advantageously predict the plausibility of progression into end-stage renal disease in advance.

## Description

### [Technical Field]

The present invention relates to a composition for diagnosing kidney disease using a urine sample, and more particularly to a composition for a urine test for diagnosing kidney disease including an agent that specifically detects amphiregulin from a urine sample, a kit therefor, and a method of diagnosing kidney disease using the composition.

### [Background Art]

With large changes in lifestyle centered on diet and population ageing in recent years, the incidence of lifestyle-related diseases such as hypertension or diabetes has increased, and accordingly, the risk of kidney disease, and further, renal failure is increasing.

The kidneys are an important organ to maintain homeostasis of the internal environment through the excretion of waste products and regulation of body fluids, electrolytes, and acid-base balance. The kidneys control the concentrations of various compounds in the blood, including hydrogen, sodium, potassium, and silicon, and excrete waste products in the urine. Any decline in kidney function may interfere with the body's ability to sufficiently remove metabolites from the blood and disrupt the body's electrolyte balance. In the case of decline or failure of kidney function, symptoms thereof are fatal.

Chronic kidney disease is a state in which the kidney function gradually decreases unidirectionally (irreversibly) and also in which homeostasis of the body is not maintained. Chronic kidney disease is known to be caused by diabetic renal failure, chronic glomerulonephritis, malignant nephrosclerosis, polycystic kidney disease, etc. All kidney diseases involve renal fibrosis, eventually leading to end-stage renal disease. In particular, since chronic kidney function decline is deeply related to the progression of renal fibrosis, it is thought that inhibition of progression of fibrosis may lead to inhibition of progression to chronic kidney disease.

In general, renal fibrosis involves an inflammatory response due to endothelial cell disorders, and thus an overproduced extracellular matrix (ECM) is caused by fibrosis. For example, in glomerulosclerosis, it is caused by a protoglomerular endothelial cell disorder, cytokines such as chemokine growth factors are secreted, and monocytes or macrophages cause an inflammatory response. Subsequently, activation, proliferation, and transformation of mesangial cells occur, and an excessive amount of extracellular matrix is produced in extracellular-matrix-producing cells such as mesangial cells, resulting in fibrosis and thus glomerulosclerosis.

In particular, diabetic renal failure is the leading cause of chronic kidney disease, and is one of the serious complications of diabetes. The hallmark of diabetic renal failure is the proliferation of glomerular mesangial cells, which is mainly associated with increased accumulation of ECM proteins such as type I and type IV collagen, fibronectin, laminin, and the like (Verena Klemis et al., Kidney International (2017) 91, 1374-1385) .

When chronic kidney disease worsens and progresses until the kidneys fail to function completely, it leads to end-stage renal disease (ESRD). Recovery from end-stage renal disease is impossible, and patients with end-stage renal disease must either undergo dialysis (hemodialysis or peritoneal dialysis) or obtain a new kidney through transplantation in order to restore the kidney function thereof.

Meanwhile, it is known that amphiregulin binds to the epidermal growth factor receptor (EGFR) and activates the EGFR pathway and is involved in cell proliferation, and it has been disclosed that amphiregulin-specific siRNA inhibits the expression of amphiregulin, which has therapeutic effects in certain types of breast cancer (Cancer Res. 2008; 68: 225-2265). In addition, it has been reported that it is possible to inhibit cell infiltration in inflammatory breast cancer using shRNA for amphiregulin (J. Cell Physiol. 2011 226(10):2691-2701), and also that inhibition of amphiregulin expression using amphiregulin-specific shRNA suppresses pulmonary artery remodeling in mice exposed to cigarette smoke. It has been reported that amphiregulin is associated with airway smooth muscle (ASM) hyperplasia and angiogenesis, and particularly, airway remodeling in asthmatic patients is promoted and the oversecreted epidermal growth factor (EGF) and amphiregulin are involved in tissue remodeling following acute asthma.

As diagnostic methods using amphiregulin, a method of diagnosing chorioamnionitis (WO 2008-029664) and a method of diagnosing inflammatory disease (US 2006-0286586) are disclosed, but a study for diagnosing kidney disease using amphiregulin has not been progressed as of yet.

Accordingly, the present inventors have recognized the importance of early diagnosis of kidney disease by paying attention to the fact that recovery is not easy when kidney disease progresses to an advanced stage, and ascertained that, during searching for methods of easily diagnosing kidney disease from urine that may be collected without invasive action, when the concentration of amphiregulin in urine is checked, it is possible to easily diagnose kidney disease and predict the progression to end-stage renal disease, thus culminating in the present invention.

### [Disclosure]

It is an object of the present invention to provide a composition for diagnosing kidney disease capable of easily early-diagnosing kidney disease from a urine sample, a kit therefor, a method of providing information for diagnosis, and a method of providing information for predicting the likelihood of progression to end-stage renal disease.

In order to accomplish the above object, the present invention provides a composition for diagnosing kidney disease including an agent that specifically detects amphiregulin or mRNA thereof.

In addition, the present invention provides a kit for diagnosing kidney disease including the composition.

In addition, the present invention provides a method of providing information for diagnosing kidney disease, including:
(a) taking a urine sample from a subject;
(b) detecting amphiregulin in the urine sample; and
(c) determining that the subject has kidney disease when the amount of amphiregulin that is detected in the urine sample is 5 pg/mgCr or more.

In addition, the present invention provides a method of providing information for predicting the likelihood of progression to end-stage renal disease, including:
(a) taking a urine sample from a patient suffering from kidney disease;
(b) measuring the amount of amphiregulin in the urine sample; and
(c) determining that the patient is likely to progress to end-stage renal disease when the amount of amphiregulin that is measured is 30 pg/mgCr or more.

In addition, the present invention provides the use of the composition including an agent that specifically detects amphiregulin or amphiregulin mRNA for the diagnosis of kidney disease.

In addition, the present invention provides the use of the composition including an agent that specifically detects amphiregulin for the manufacture of a reagent for the diagnosis of kidney disease.

In addition, the present invention provides a method of diagnosing kidney disease, including:
(a) taking a urine sample from a subject;
(b) detecting amphiregulin in the urine sample; and
(c) determining that the subject has kidney disease when the amount of amphiregulin that is detected in the urine sample is 5 pg/mgCr or more.

In addition, the present invention provides a method of predicting the likelihood of progression to end-stage renal disease, including:
(a) taking a urine sample from a patient suffering from kidney disease;
(b) measuring the amount of amphiregulin in the urine sample; and
(c) determining that the patient is likely to progress to end-stage renal disease when the amount of amphiregulin that is measured is 30 pg/mgCr or more.

### [Description of Drawings]

FIG. 1 shows the results of measurement of the mRNA expression levels of amphiregulin in kidney and lung tissues of a normal animal (Normal) and an animal model of lung fibrosis (BLM);
FIG. 2 shows the results of measurement of the mRNA expression levels of collagen 1 in the kidney and lung tissues of a normal animal (Normal) and an animal model of lung fibrosis (BLM);
FIG. 3 shows the results of measurement of the mRNA expression levels of fibronectin in the kidney and lung tissues of a normal animal (Normal) and an animal model of lung fibrosis (BLM);
FIG. 4 shows the results of measurement of the amphiregulin/creatinine ratio through ELISA in urine of a normal animal (Normal) and an animal model of lung fibrosis (BLM);
FIG. 5 shows the results of measurement of the albumin/creatinine ratio through ELISA in urine of a normal animal (Normal) and an animal model of lung fibrosis (BLM);
FIG. 6a shows the results of H&E staining or MT staining of the lung tissue of a normal animal (Normal) and an animal model of lung fibrosis (BLM);
FIG. 6b shows the results of H&E staining or MT staining of the kidney tissue of a normal animal (Normal) and an animal model of lung fibrosis (BLM);
FIG. 7 is a graph showing blood urea nitrogen in the serum samples of a normal animal (Normal) and an animal model of lung fibrosis (BLM);
FIG. 8 is a graph showing the creatinine levels in the serum samples of a normal animal (Normal) and an animal model of lung fibrosis (BLM);
FIG. 9 is graphs comparing the concentrations of amphiregulin in the serum (A) and urine (B) of patients diagnosed with IgA (immunoglobulin A) nephropathy and diabetic nephropathy having severe renal pathological findings compared to minor glomerular change among patients who underwent a renal biopsy;
FIG. 10 is graphs showing the comparison of urine amphiregulin concentrations depending on the severity in biopsy findings related to renal fibrosis in the patient group of FIG. 9 (A: interstitial fibrosis, B: intimal thickening, C: tubular atrophy, C: mesangial expansion);

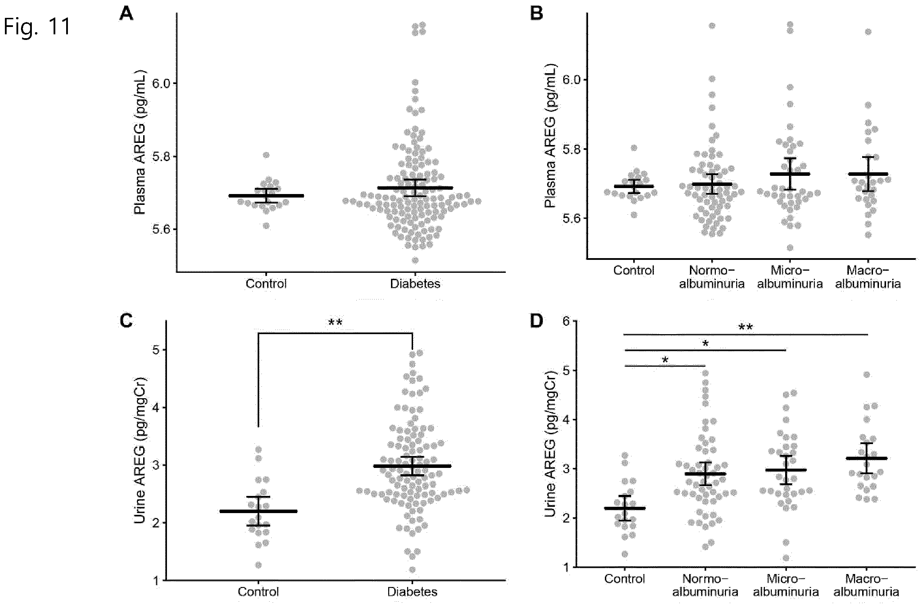

FIG. 11A shows the results of analysis of the concentration of amphiregulin in plasma in a normal control group and a group of patients with type 2 diabetes, FIG. 11B shows the results of analysis of the concentration of amphiregulin in plasma in three groups into which the group of patients with type 2 diabetes is additionally divided depending on the level of albuminuria, FIG. 11C shows the results of analysis of the concentration of amphiregulin in urine in the normal control group and the group of patients with type 2 diabetes, and FIG. 11D shows the results of analysis of the concentration of amphiregulin in urine in three groups into which the group of patients with type 2 diabetes is additionally divided depending on the level of albuminuria;
FIG. 12 shows results confirming the amount of amphiregulin in urine depending on the stage of chronic kidney disease in a group of patients who underwent a renal biopsy; and
FIG. 13 is a Kaplan-Meier survival curve graph comparing the progression to end-stage renal disease depending on the concentration of amphiregulin in a group of patients who underwent a renal biopsy, in which urine amphiregulin concentrations were divided into tertiles, which were designated as T1, T2, and T3 from the lowest value.

### [Mode for Invention]

Unless otherwise defined, all technical and scientific terms used herein have the same meanings as those typically understood by those skilled in the art to which the present invention belongs. In general, the nomenclature used herein is well known in the art and is typical.

In the present invention, a renal biopsy was performed on patients suspected of having kidney damage due to various diseases, confirming that amphiregulin in urine of this patient group reflects the extent of fibrosis inside the kidneys.

Accordingly, an aspect of the present invention pertains to a composition for diagnosing kidney disease including an agent that specifically detects amphiregulin or mRNA thereof.

In the present invention, the agent is able to specifically detect amphiregulin from a urine sample.

In the present invention, the agent that specifically detects amphiregulin may be an amphiregulin-specific antibody or aptamer, but is not limited thereto, and any agent may be used without limitation, so long as it is able to detect the presence or absence of amphiregulin mRNA or protein in the urine sample or to measure the amount thereof. In an embodiment of the present invention, an ELISA method to which the amphiregulin-specific antibody is applied was used, but a PCR method using a primer and/or a PNA probe for measuring the amount of amphiregulin mRNA may also be used.

In the present invention, the "agent that specifically detects amphiregulin" is a molecule that may be used for detection of a marker by measuring the expression level of a biomarker (i.e. amphiregulin mRNA or protein), the expression of which is changed due to abnormal kidney function. The expression level of the corresponding biomarker may be determined by measuring the expression level of the marker mRNA or protein.

In the present invention, the "antibody" is a term known in the art and refers to a specific protein molecule directed to an antigenic site. For the purposes of the present invention, the antibody refers to an antibody that specifically binds to the biomarker protein of the present invention, and after obtaining a protein that is encoded by the marker gene by cloning each gene into an expression vector according to a typical method, such an antibody may be produced from the protein thus obtained through a typical method. The antibody also includes a partial peptide that may be made from the protein, and the partial peptide of the present invention includes at least 7 amino acids, preferably 9 amino acids, more preferably 12 or more amino acids.

The form of the antibody of the present invention is not particularly limited, and a polyclonal antibody, a monoclonal antibody, or a part thereof having antigen-binding properties is also included in the antibody of the present invention, and all immunoglobulin antibodies are included. Furthermore, the antibody of the present invention includes a special antibody such as a humanized antibody, etc.

The antibody used for the detection of the marker for diagnosing kidney function of the present invention includes not only a complete form having two full-length light chains and two full-length heavy chains, but also a functional fragment of an antibody molecule. The functional fragment of the antibody molecule is a fragment having at least an antigen-binding function, and examples thereof include Fab, F(ab'), F(ab')2, and Fv.

In the present invention, "measuring the expression level of a protein" is a process of confirming the presence and expression level of a kidney function abnormality marker protein in the biosample of a subject in order to diagnose kidney disease, and, for example, the amount of the protein may be measured by detecting the antigen-antibody complex using an antibody that specifically binds to the corresponding marker protein. Specific examples of the analysis method include Western blot, ELISA (enzyme-linked immunosorbent assay), radioimmunoassay (RIA), radial immunodiffusion, Ouchterlony immunodiffusion, rocket immunoelectrophoresis, tissue immunostaining, immunoprecipitation assay, complement fixation assay, FACS, protein chip assay, etc., but are not limited thereto.

In the present invention, "diagnosis" means identifying the presence or characteristics of a pathological condition. For the purposes of the present invention, the diagnosis may be interpreted as determining whether or not kidney disease occurs.

As used herein, the term "marker for diagnosis", "marker for performing diagnosis", or "diagnostic marker" refers to a material capable of distinguishing a sample in which abnormal kidney function has occurred or is likely to occur from a sample of a healthy subject. A sample from a subject with abnormal kidney function includes a polypeptide, nucleic acid (e.g. mRNA, etc.), or protein, which shows increased expression of the diagnostic marker (i.e. amphiregulin) when compared with a sample from a healthy subject.

The protein or gene information of the biomarker for diagnosing kidney disease provided in the present invention may be easily obtained through a known genetic database. For example, the amino acid sequence of the biomarker protein amphiregulin is registered in NCBI (National Center for Biotechnology Information), a known genetic database, and is as follows.

SEQ ID NO: 1

MRAPLLPPAPVVLSLLILGSGHYAAGLDLNDTYSGKREPFSGDHSADGFEV

TSRSEMSSGSEISPVSEMPSSSEPSSGADYDYSEEYDNEPQIPGYIVDDSVRVEQV

VKPPQNKTESENTSDKPKRKKKGGKNGKNRRNRKKKNPCNAEFQNFCIHGECKYIE

HLEAVTCKCQQEYFGERCGEKSMKTHSMIDSSLSKIALAAIAAFMSAVILTAVAVI

TVQLRRQYVRKYEGEAEERKKLRQENGNVHAIA

In the present invention, the amphiregulin may be represented by the amino acid sequence of SEQ ID NO: 1, but is not limited thereto.

In the present invention, kidney disease may be selected from the group consisting of renal fibrosis, nephrotic syndrome, kidney cancer, chronic kidney disease, diabetic nephropathy, acute pyelonephritis, acute renal failure, end-stage renal disease, hypertensive kidney disease, Reye's syndrome, gout, Sjogren's syndrome, Behcet's disease, lupus, candidiasis, hemorrhagic fever with renal syndrome, leptospirosis, legionellosis, autosomal dominant polycystic kidney disease, and hydronephrosis, but is not limited thereto.

Another aspect of the present invention pertains to a kit for diagnosing kidney disease including the composition.

The kit of the present invention is capable of detecting the marker by measuring the expression level of the marker protein for diagnosing kidney disease. The kit for diagnosing kidney disease of the present invention may include not only an antibody that selectively recognizes a marker in order to measure the expression level of a marker for diagnosing kidney function, but also one or more other component composition solutions or devices suitable for the analysis method.

In a specific embodiment, the kit for measuring the expression level of a protein for diagnosing kidney disease in the present invention may include a substrate, an appropriate buffer solution, a secondary antibody labeled with a chromogenic enzyme or fluorescent material, and a chromogenic substrate, necessary for immunological detection of the antibody. Examples of the substrate may include a nitrocellulose membrane, a 96-well plate synthesized from polyvinyl resin, a 96-well plate synthesized from polystyrene resin, and a glass slide, the chromogenic enzyme may be peroxidase or alkaline phosphatase, the fluorescent material may be FITC, RITC, or the like, and the chromogenic substrate solution may be ABTS (2,2'-azino-bis-(3-ethylbenzothiazoline-6-sulfonic acid), OPD (o-phenylenediamine), TMB (tetramethyl benzidine), or the like.

In the present invention, the kit may be a protein chip kit or an immunodiagnostic kit, but is not limited thereto.

Still another aspect of the present invention pertains to a method of providing information for diagnosing kidney disease, including:
(a) taking a urine sample from a subject;
(b) detecting amphiregulin in the urine sample; and
(c) determining that the subject has kidney disease when the amount of amphiregulin that is detected in the urine sample is 5 pg/mgCr or more.

In the present invention, when the amount of amphiregulin that is detected in the urine sample is 5 pg/mgCr or more, preferably 6 pg/mgCr or more, more preferably 6.3 pg/mgCr or more, most preferably 6.5 pg/mgCr or more, the subject may be determined to have kidney disease.

In the present invention, the detecting the amphiregulin may be performed through an amphiregulin-specific binding reaction using an amphiregulin-specific antibody or aptamer, but is not limited thereto.

Yet another aspect of the present invention pertains to a method of detecting amphiregulin from a urine sample of a subject in order to provide information necessary for diagnosing kidney disease.

Specifically, the expression of a gene may be detected at the protein level, and isolation of the protein from a biosample may be performed using a known process.

As used herein, the term "subject" refers to an animal having kidneys among body organs in living things existing in nature, preferably a mammal, and more preferably a human. Examples of the mammal include a rat (rat, mouse, etc.), rabbit, horse, cow, sheep, dog, cat, monkey, and human, but the type of the subject of the present invention is not limited to the above example.

In the present invention, the sample for detecting amphiregulin may include a tissue, cell, whole blood, serum, plasma, saliva, sputum, cerebrospinal fluid, or urine, in which the mRNA or protein expression level of amphiregulin differs between a normal person and a subject with kidney disease. In particular, a urine sample is preferable.

In the present invention, examples of the analysis method for detecting a marker protein may include Western blot, ELISA, radioimmunoassay, radial immunodiffusion, Ouchterlony immunodiffusion, rocket immunoelectrophoresis, tissue immunostaining, immunoprecipitation assay, complement fixation assay, FACS, protein chip assay, and the like, but are not limited thereto. Through the above analysis methods, the amount of an antigen-antibody complex that is formed in a normal control group may be compared with the amount of an antigen-antibody complex that is formed in a subject suspected of having kidney disease, and whether kidney disease actually occurs in the subject suspected of having kidney disease may be predicted or confirmed by determining whether there is a significant increase in the protein expression level in the marker gene for diagnosing kidney disease. The antigen-antibody complex is a product resulting from binding of a marker protein for diagnosing kidney disease and an antibody specific thereto, and the amount of the antigen-antibody complex that is formed may be quantitatively measured through the magnitude of a signal of a detection label.

The protein expression level is preferably measured using an ELISA method. Examples of ELISA may include various ELISA methods, such as direct ELISA using a labeled antibody recognizing an antigen attached to a solid support, indirect ELISA using a labeled antibody recognizing a capture antibody in an antibody complex recognizing an antigen attached to a solid support, direct sandwich ELISA using another labeled antibody recognizing an antigen in an antibody-antigen complex attached to a solid support, indirect sandwich ELISA using a labeled secondary antibody recognizing an antibody reacted with another antibody recognizing an antigen in an antibody-antigen complex attached to a solid support, and the like. More preferably, the protein expression level is detected using a sandwich ELISA method in a manner of attaching an antibody to a solid support, reacting the sample, and then attaching a labeled antibody recognizing the antigen of the antigen-antibody complex to enzymatically develop color or attaching a labeled secondary antibody to the antibody recognizing the antigen of the antigen-antibody complex to enzymatically develop color. By measuring the extent of formation of a complex between an antibody and a marker protein for diagnosing kidney disease, it is possible to determine whether kidney disease occurs.

Also, it is preferred to perform Western blot using one or more antibodies for the marker for diagnosing kidney disease. For example, total protein is isolated from a sample, and the protein is sorted according to size through electrophoresis and then transferred to a nitrocellulose membrane to react with an antibody. The amount of the antigen-antibody complex that is formed is measured using a labeled antibody, such that the amount of the protein produced by gene expression is measured, thus determining whether there is abnormal kidney function. This detection method includes a method of examining the expression level of the marker gene in the control group and the expression level of the marker gene in cells with abnormal kidney function. The protein level may be represented as an absolute (e.g. µg/ml) or relative (e.g. relative intensity of signal) difference in the marker protein described above.

Also, it is preferred to use a protein chip in which one or more antibodies for the marker for diagnosing kidney disease are arranged at a predetermined position on a substrate and immobilized at a high density. For example, in a method of analyzing a sample using a protein chip, a protein is isolated from the sample, the isolated protein is hybridized with the protein chip to form an antigen-antibody complex, which is then read, whereby the presence or expression level of the protein is measured, making it possible to determine whether there is abnormal kidney function.

Through the above detection methods, the protein expression level in the normal control group is compared with the gene expression level in the subject suspected of having kidney disease, making it possible to confirm or predict the abnormal kidney function of the patient suspected of having kidney disease. Specifically, the expression level of the marker of the present invention in a urine sample of a subject suspected of having kidney disease and the expression level of the marker of the present invention in a urine sample of a healthy subject are measured and compared, after which, when the expression level of the marker of the present invention is determined to be significantly increased compared to that of the healthy subject, it is predicted that the subject has kidney disease.

Meanwhile, according to the experimental results of the present invention, amphiregulin in urine reflects the basal kidney function well, so it is possible to predict the extent of kidney damage at the time of measurement, and also to predict the progression of kidney damage in the future, as well as at the time of measurement, through analysis using follow-up data. Here, amphiregulin may be applied as a biomarker predicting end-stage renal disease in kidney disease patients by measuring the amount thereof in urine.

Accordingly, still yet another aspect of the present invention pertains to a method of providing information for predicting the likelihood of progression to end-stage renal disease, including:
(a) taking a urine sample from a patient suffering from kidney disease;
(b) measuring the amount of amphiregulin in the urine sample; and
(c) determining that the patient is likely to progress to end-stage renal disease when the amount of amphiregulin that is measured is 30 pg/mgCr or more.

In the present invention, when the amount of amphiregulin that is measured is 30 pg/mgCr or more, preferably 35 pg/mgCr or more, more preferably 39 pg/mgCr or more, most preferably 39.9 pg/mgCr or more, it may be determined that the patient is likely to progress to end-stage renal disease.

In the present invention, the patient suffering from kidney disease may be a patient with chronic kidney disease, but is not limited thereto.

In the present invention, in step (c), the likelihood of progression to end-stage renal disease, particularly the probability of progression to end-stage renal disease within 2 years after measurement of urine amphiregulin is determined to be 50%, preferably 60% or more, but is not limited thereto.

A further aspect of the present invention pertains to the use of the composition including an agent that specifically detects amphiregulin for the diagnosis of kidney disease.

Still a further aspect of the present invention pertains to the use of the composition including an agent that specifically detects amphiregulin for the manufacture of a reagent for the diagnosis of kidney disease.

Yet a further aspect of the present invention pertains to a method of diagnosing kidney disease, including:
(a) taking a urine sample from a subject;
(b) detecting amphiregulin in the urine sample; and
(c) determining that the subject has kidney disease when the amount of amphiregulin that is detected in the urine sample is 5 pg/mgCr or more.

Still yet a further aspect of the present invention pertains to a method of predicting the likelihood of progression to end-stage renal disease, including:
(a) taking a urine sample from a patient suffering from kidney disease;
(b) measuring the amount of amphiregulin in the urine sample; and
(c) determining that the patient is likely to progress to end-stage renal disease when the amount of amphiregulin that is measured is 30 pg/mgCr or more.

A better understanding of the present invention may be obtained through the following examples. These examples are merely set forth to illustrate the present invention, and are not to be construed as limiting the scope of the present invention.

### Example 1. Verification of amphiregulin expression in urine sample from animal model of lung fibrosis with normal kidney function

In order to determine whether there is abnormal kidney function using a urine sample of an animal model of lung fibrosis, an experiment for detecting amphiregulin in a urine sample was conducted.

### 1.1 Animal experimental method

In order to construct an animal model of lung fibrosis, 15 units of C57BL/6 (Mouse) bleomycin (BLM) (B5507, Sigma, USA) was dissolved in 5 ml of PBS to make 3 Unit/ml, after which 50 µl thereof was administered to C57BL/6 (Mouse) through an intratracheal instillation route in order to become 1.5 Unit/kg based on the mouse body weight. 14 days after construction of the animal model with bleomycin-induced lung fibrosis, the following parameters were measured: body weight, albumin/creatinine ratio in urine, and serum creatinine. Additionally, histological analysis was performed on lung tissue and kidney tissue. In addition, fibronectin and collagen 1 were analyzed to determine whether fibrosis occurred in lung tissue and kidney tissue. Also, gene expression analysis was performed to determine how much amphiregulin was expressed in each tissue.

### 1.2 RNA isolation and analysis of fibrosis gene and amphiregulin gene expression

The lung and kidney tissues of the sacrificed mice were obtained and the tissues were ground using a homogenizer. Total RNA was extracted using an RNA extraction kit (AccuPrep Cell total RNA extraction kit, BIONEER, Korea). 1 µg of the extracted RNA was quantified using a spectrophotometer. cDNA was synthesized using RNA reverse transcriptase (AccuPower^{®} RocketScript^{™} RT Premix with oligo (dT)20, Bioneer, Korea) according to the manufacturer's instructions. Using the synthesized cDNA as a template, the relative expression levels of total mRNA in each group were analyzed through real-time qPCR using the SYBR green method in the following manner. In each well of a 96-well plate, the synthesized cDNA was diluted 5-fold with distilled water, and for analysis of the mRNA expression level of each gene, 3 µl of the diluted cDNA, 25 µl of AccuPower^{®} GreenStar^{™} (Korea), 19 µl of distilled water, and 3 µl of each gene-specific qPCR primer (10 pmole/µl, BIONEER, Korea) were added to make a mixed solution. Also, in order to normalize fibronectin, collagen 1, and amphiregulin genes, RPL13A, which is a housekeeping gene (hereinafter referred to as an HK gene), was used as a standard gene. Primer sequences for identifying gene expression in each tissue are as follows.

**[Table 1]**

| Primer sequence | | |
|---|---|---|
| | Forward Primer | Reverse Primer |
| RPL13A | 5'-CGATAGTGCATCTTGGCCTTT-3' (SEQ ID NO: 2) | 5'-CCTGCTGCTCTCAAGGTTGTT-3' (SEQ ID NO: 3) |
| Amphiregulin | 5'-GAGGCTTCGACAAGAAAACG-3' (SEQ ID NO: 4) | 5'-ACCAATGTCATTTCCGGTGT-3' (SEQ ID NO: 5) |
| Fibronectin | 5'-TGGTGGCCACTAAATACGAA-3' (SEQ ID NO: 6) | 5'-GGAGGGCTAACATTCTCCAG-3' (SEQ ID NO: 7) |
| Collagen I | 5'-TCATCGTGGCTTCTCTGGTC-3' (SEQ ID NO: 8) | 5'-GACCGTTGAGTCCGTCTTTG-3' (SEQ ID NO: 9) |

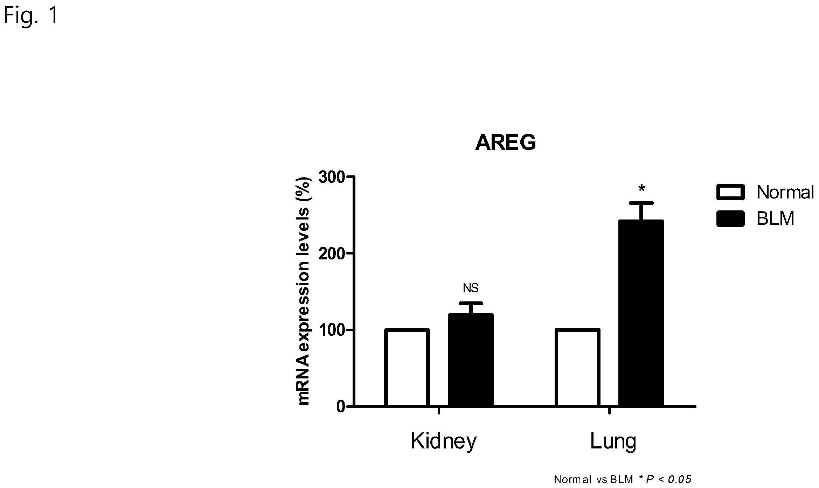

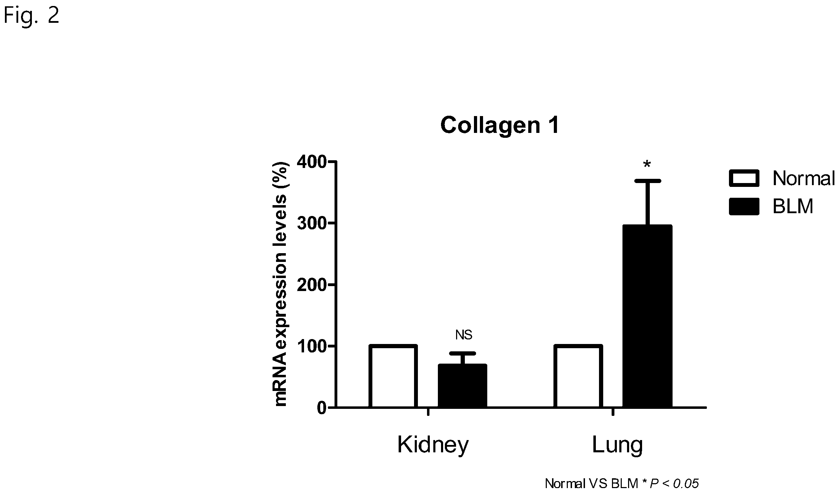

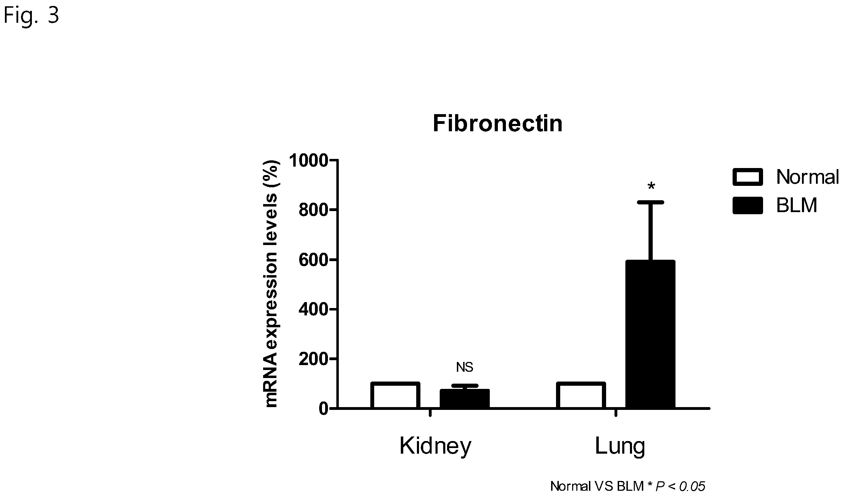

Consequently, as shown in FIG. 1, there was no difference in gene expression between bleomycin-induced mice and healthy mice in the kidney tissue, whereas the difference in AREG was statistically significantly increased in bleomycin-induced mice in the lung tissue. Also, as shown in FIGS. 2 and 3, collagen 1 and fibronectin, which are fibrosis markers, were measured in order to determine whether fibrosis progressed in each tissue, confirming that there was no difference in expression of collagen 1 and fibronectin between healthy mice and bleomycin-induced mice in the kidney tissue, but that the expression thereof was statistically significantly increased in mice with bleomycin-induced lung fibrosis in the lung tissue compared to healthy mice. Therefore, in the mice with bleomycin-induced lung fibrosis, it was confirmed by gene expression analysis that fibrosis was induced only in the lung tissue, but not in the kidney tissue.

### 1.3. Detection of amphiregulin and albumin/creatinine ratio in urine sample

### 1-3-1. Mouse Amphiregulin ELISA method

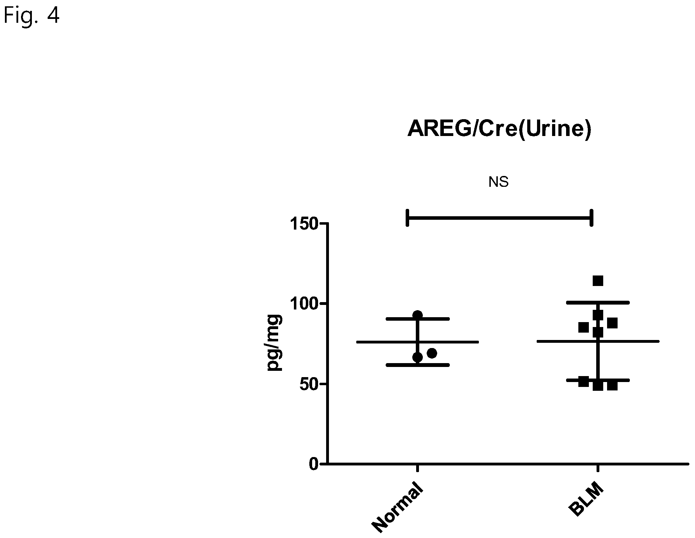

Mouse Amphiregulin Duoset ELISA (DY989, R&D Systems, USA) was used to detect amphiregulin in mouse urine. 100 µl of a mouse capture amphiregulin antibody (DY989, R&D Systems, USA) was added at a concentration of 800 ng/ml to each well to coat the same. Thereafter, 100 µl of 1% BSA was added to each well, followed by reaction at room temperature for 1 hour to remove non-specific reaction other than the sample-antibody reaction. Each well was washed three times using PBST containing 0.05% Tween20 as a washing buffer. Next, 100 µl of mouse urine was added to each well of the 96-well plate that was coated, followed by reaction at room temperature for 2 hours. After reaction, urine samples were removed from each well and each well was then washed three times using PBST containing 0.05% Tween20 as a washing buffer. 100 µl of a detection antibody (DY989, R&D Systems, USA) was added at a concentration of 25.0 ng/ml to each well, followed by reaction at room temperature for 2 hours. After reaction, the detection antibody solution was removed and each well was then washed three times with PBST. 100 µl of a streptavidin-HRP (DY989, R&D Systems, USA) secondary antibody diluted at a ratio of 1:200 was added to each well, followed by reaction at room temperature for 20 minutes. After reaction, each well was washed three times for 5 minutes using PBST. 100 µl of 1-step Ultra TMB (34028, Thermo, USA) was added to each well, followed by reaction at room temperature. Then, 100 µl of sulfuric acid (H₂SO₄) was added to each well to stop the reaction, after which the absorbance of each well was measured at a wavelength of 450 nm using a spectrophotometer. The quantitative value of each sample was calculated by creating an equation for the standard curve using the measured absorbance values. Consequently, the amphiregulin/creatinine ratio corrected for the creatinine value did not show a great difference between healthy mice and lung-fibrosis-induced mice. In the lung fibrosis model with normal kidney function, there was no great difference in amphiregulin/creatinine levels in urine compared to healthy mice (FIG. 4).

### 1-3-2. Detection of albumin/creatinine ratio in mouse urine

The experiment was carried out using an albumin ELISA kit (Excocell, Phila, PA19103, Cat:1011). In order to measure albumin, a urine sample was diluted at a ratio of 1:13 using an NHE buffer (Excocell, Phila, PA19103, Cat:1011), and 50 µl thereof was added to each well. 50 ul of a primary Ab (Excocell, Phila, PA19103, Cat:1011) was added to each well, followed by reaction at room temperature for 1 hour. Then, each well was washed ten times using distilled water, and 100 µl of a 2-color developer (Excocell, Phila, PA19103, Cat: 1011) was added to each well, followed by reaction at room temperature for 20 minutes. 100 µl of a 2-color stopper (Excocell, Phila, PA19103, Cat: 1011) was added to each well to stop the reaction, and the absorbance was measured at 450 nm using a spectrophotometer. The quantitative value of each sample was calculated by creating a calculation equation for the standard curve using the measured absorbance values.

The reason for measuring creatinine in urine is to compensate for a change in the amount of urine amphiregulin at the time of measurement depending on the concentration of urine. Therefore, in order to measure creatinine as follows, a creatinine ELISA kit (Excocell, Phila, PA19103, Cat:1012) was used. A urine sample was diluted with distilled water at a ratio of 1:10 and added to each well. Then, a picrate solution (Excocell, Phila, PA19103, Cat:1012) was prepared, and 100 µl thereof was added to each well, followed by reaction at room temperature, after which the absorbance was primarily measured at 500 nm using a spectrophotometer, after which 100 µl of an acid reagent (Excocell, Phila, PA19103, Cat:1012) was added to each well, and the absorbance was secondarily measured after 5 minutes. The final result value was obtained by subtracting the secondary absorbance value from the primary absorbance value. The quantitative value of each sample was calculated by creating a calculation equation for the standard curve using the absorbance values measured at creatinine standard concentrations of 10 mg/dL, 3 mg/dL, and 1 mg/dL.

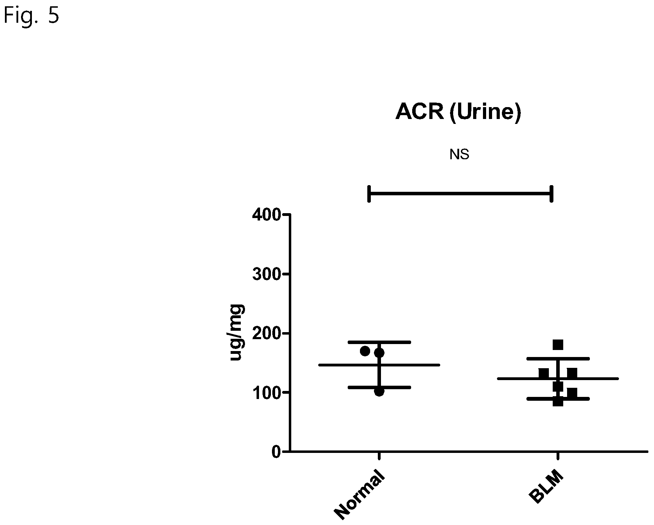

Also, the albumin/creatinine ratio did not show a great difference between the lung-fibrosis-induced mice and the healthy mice (FIG. 5), indicating that the kidney function was normally maintained in the lung-fibrosis-induced mice.

### 1.4. Histopathological analysis

Immunohistochemical staining was performed for histopathological verification of lung tissue and kidney tissue in the constructed animal model. The animals of each group were sacrificed, and paraffin sections were prepared through tissue fixation, water washing, dehydration, clearing, infiltration, embedding, and sectioning. The paraffin sections were cut into thin slices with a slicer, and the tissue was adhered to the slide. The adhered tissue was deparaffinized, hydrated, washed with water, stained, dehydrated, cleared, and coverslipped, followed by hematoxylin and eosin staining and Masson's trichrome staining. Then, tissue staining was performed by the KPNT analysis agency.

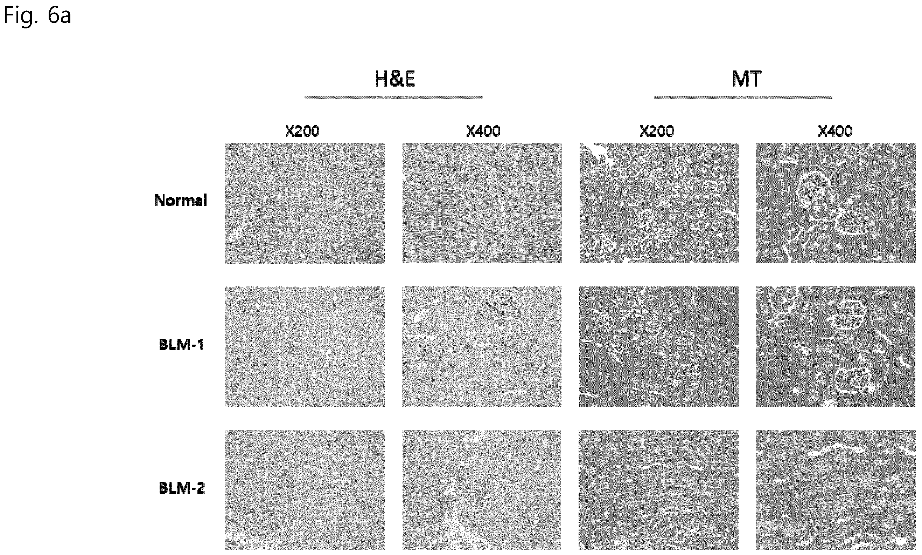

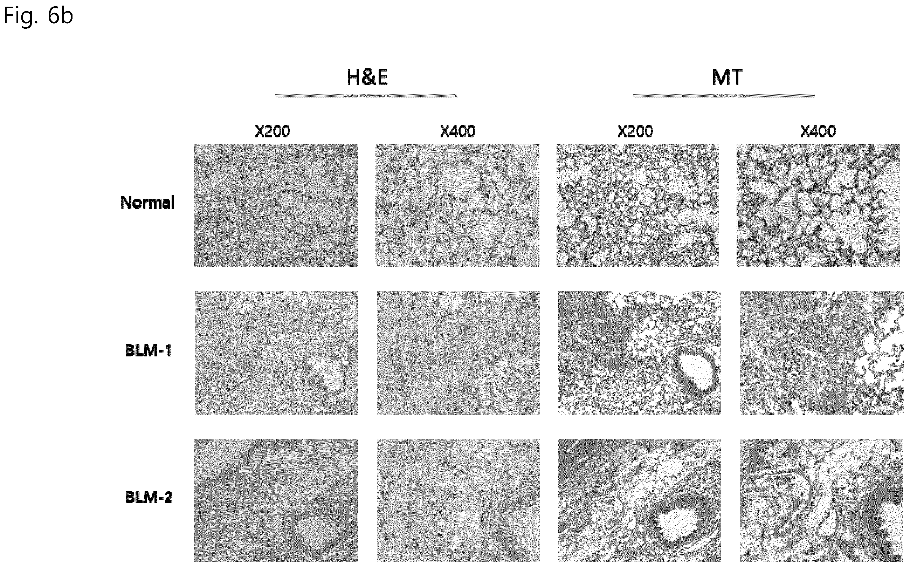

Consequently, it was found that lung-fibrosis-induced mice had more differentiated lung tissue fibrosis cells than healthy mice, and based on the results of analysis of the expression level of collagen 1 through MT staining, it was confirmed that more collagen was deposited in the lung-fibrosis-induced mice than in the healthy mice (FIG. 6A). However, in the kidney tissue, no significant difference was observed between the healthy mice and the lung-fibrosis-induced mice but a similar tissue state was maintained, and no deposition of collagen 1 was observed in the lung-fibrosis-induced mice (FIG. 6B).

### 1-5. Measurement of blood urea nitrogen and creatinine levels

When kidney function deteriorates, materials excreted through the kidneys accumulate in the body. BUN and creatinine are the most representative materials, and it is known that the concentrations of these materials reflect kidney function well. In order to confirm kidney function, blood urea nitrogen (BUN) and creatinine levels in serum samples were measured.

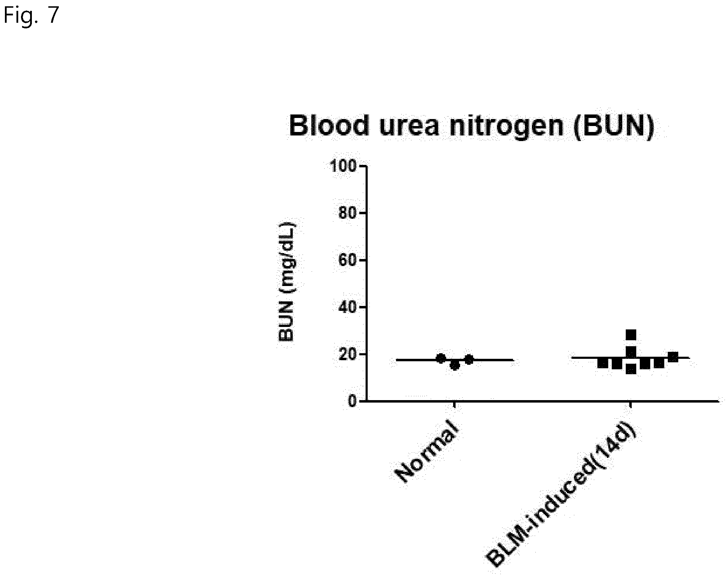

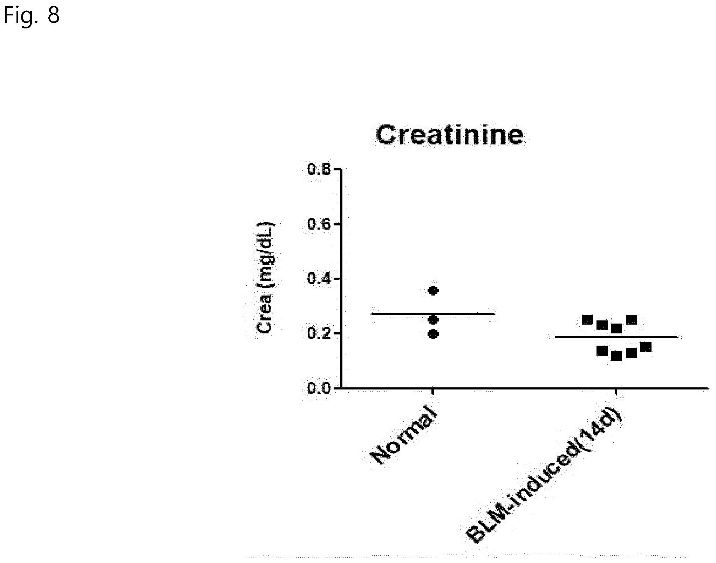

As shown in FIG. 7, it was confirmed that there was no statistically significant difference when the blood urea nitrogen of the bleomycin-induced mice was compared with that of the healthy mice. In addition, as shown in FIG. 8, when creatinine levels were compared, there was no difference between the healthy mice and the bleomycin-induced mice. Therefore, it was confirmed that the kidney function of the animal model with bleomycin-induced lung fibrosis was maintained normally.

### Example 2. Verification of amphiregulin expression in serum and urine of patient group who underwent renal biopsy

The concentration of amphiregulin was measured through ELISA in the serum and urine of the patient group who underwent a renal biopsy. The expression of amphiregulin in the serum and urine was analyzed by comparing patients with minor glomerular change having pathological findings similar to those of normal kidneys with patients with IgA (immunoglobulin A) nephropathy and diabetic nephropathy having more severe pathological findings. In addition, based on the results of renal biopsy, the above patients were divided into four groups depending on the biopsy findings related to renal fibrosis, and the expression levels of amphiregulin in urine of these groups were compared.

### 2-1. Analysis of amphiregulin through ELISA in serum and urine samples of patients

In order to detect amphiregulin in human serum or urine, Human Amphiregulin Duoset ELISA (DY262, R&D Systems, USA) was used. Specifically, 100 µl of an amphiregulin antibody was added at a concentration of 2.00 ug/ml to each well of a 96-well plate to coat the same. Thereafter, 100 µl of 1% BSA was added to each well, followed by reaction at room temperature for 1 hour, thereby inhibiting non-specific expression other than the sample-antibody reaction. Each well was washed three times using PBST containing 0.05% Tween20 as a washing buffer. 100 µl of a patient serum or urine stock sample was added to each well, followed by reaction at room temperature for 2 hours, after which the serum or urine sample was removed from each well, and each well was washed three times using PBST containing 0.05% Tween20 as a washing buffer. Each well was washed three times using PBST, and 100 µl of a detection antibody (DY262, R&D Systems, USA) was added at a concentration of 100 ng/ml to each well, followed by reaction at room temperature for 2 hours. Thereafter, the detection antibody solution was removed, each well was washed three times with PBST, and 100 µl of a streptavidin-HRP (DY262, R&D Systems, USA) secondary antibody diluted at a ratio of 1:200 was added to each well, followed by reaction at room temperature for 20 minutes. After reaction, each well was washed three times for 5 minutes using PBST, and 100 µl of 1-step Ultra TMB (34028, Thermo, USA) was added to each well, followed by reaction at room temperature. Thereafter, 100 µl of sulfuric acid (H₂SO₄) was added to each well to stop the reaction. The absorbance of each well was measured at a wavelength of 450 nm using a spectrophotometer, and the quantitative value of each sample was calculated by creating an equation for the standard curve using the measured absorbance values.

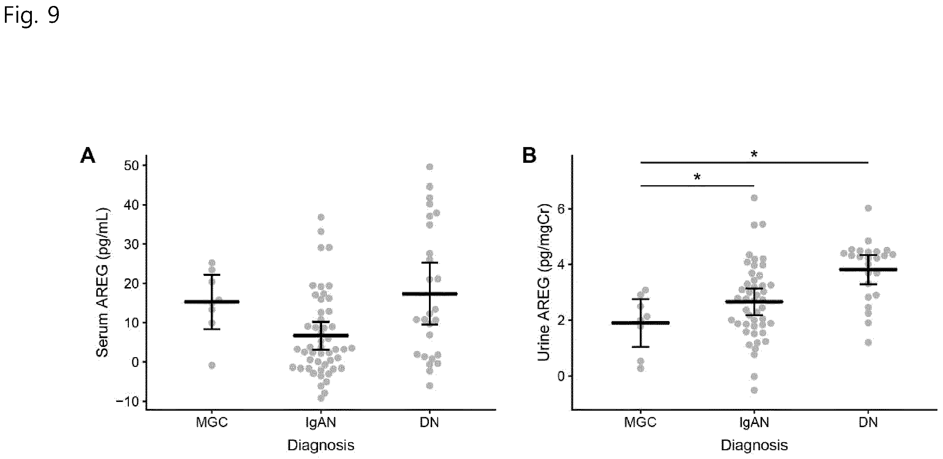

Consequently, there was no great difference in serum amphiregulin expression in patients with minor glomerular change, IgA (immunoglobulin A) nephropathy, and diabetic nephropathy. Moreover, it was found that the expression of urine amphiregulin was increased in the patients with IgA (immunoglobulin A) nephropathy and diabetic nephropathy having more severe renal pathological findings, compared to the patients with minor glomerular change (FIG. 9).

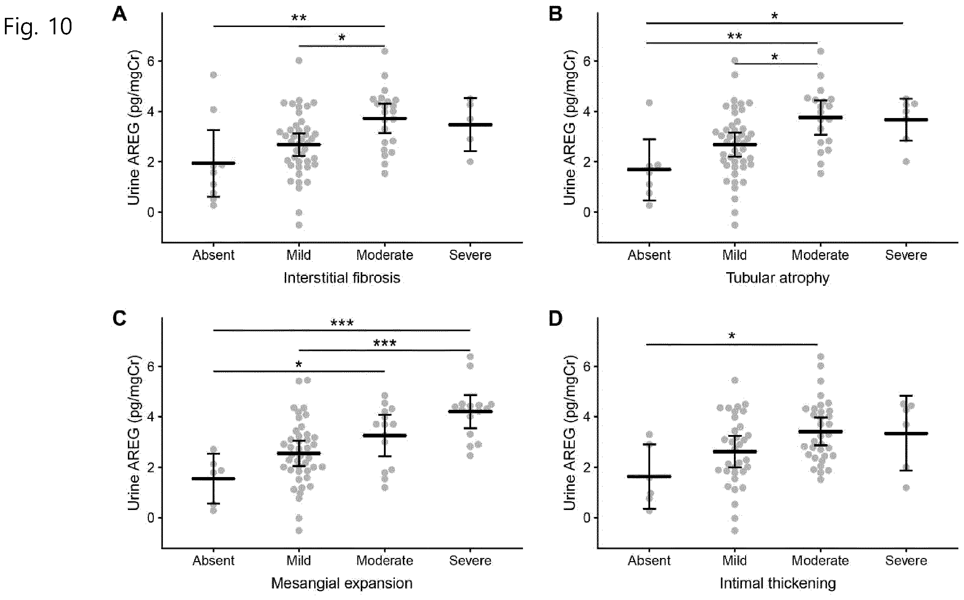

Also, the patient groups were compared and divided into no fibrosis (absent), mild, moderate, and severe groups depending on the severity in the biopsy findings related to renal fibrosis, and in the urine samples of the patients, the concentration of amphiregulin was measured. For interstitial fibrosis and intimal thickening, the expression of urine amphiregulin was significantly increased in patients in the moderate stage compared to no fibrosis (absent). For tubular atrophy and mesangial expansion, the expression of urine amphiregulin was significantly increased in patients in the moderate and severe stages compared to no fibrosis (absent). Therefore, it was found that the more severe progression of renal fibrosis in the kidney tissue further increased the expression of urine amphiregulin (FIG. 10).

Based on the results of interstitial fibrosis, corresponding to a representative measure reflecting renal fibrosis, through the above biopsy, the patients were divided into two groups, namely a patient group with no renal fibrosis (interstitial fibrosis was not observed) and a patient group with renal fibrosis (interstitial fibrosis was observed), and ROC (receiver operating characteristic) curve analysis was performed based on the concentration of urine amphiregulin. Consequently, the optimal cut-off value of amphiregulin was determined to be 6.55 pg/mg, which maximizes the sum of sensitivity and specificity.

Therefore, when the amphiregulin level in the urine sample of a subject was 6.55 pg/mgCr or more, it was confirmed that kidney disease, particularly renal fibrosis was advanced (sensitivity, 0.84; specificity, 0.78).

### Example 3. Verification of amphiregulin expression in plasma and urine of diabetic patients

The expression pattern of amphiregulin in plasma and urine samples isolated from a normal control group and patients with type 2 diabetes was confirmed. In the patients with type 2 diabetes, the severity of diabetic nephropathy can be found depending on the amount of albuminuria, so the above patients were divided into groups of normoalbuminuria, microalbuminuria, and macroalbuminuria depending on the level of albuminuria. The expression levels of amphiregulin in plasma and urine were compared for each group. ELISA was performed with the same product (DY262, R&D Systems, USA) as described in Example 2-1.

Consequently, as shown in FIG. 11, for plasma amphiregulin, there was no clear difference in concentration between the normal control group and the patients with type 2 diabetes, and there was no significant difference in concentration depending on the albuminuria group. In contrast, the urine amphiregulin concentration was remarkably increased in the patients with type 2 diabetes compared to the normal control group, and when albuminuria increased in the patients with type 2 diabetes, particularly when the severity of diabetic nephropathy increased, the urine amphiregulin concentration was also increased.

The amphiregulin-to-creatinine ratio values for the experimental groups were analyzed as follows.

**[Table 2]**

| Natural log of urine amphiregulin-to-creatinine ratio in diabetic kidney disease study cohort | |
|---|---|
| Diagnosis | Log AREG-to-creatinine ratio (pg/mgCr) |
| Normal control group | 2.08 ± 0.55 |
| Type 2 diabetes | 2.98 ± 0.76 |

**[Table 3]**

| Natural log of urine amphiregulin-to-creatinine ratio in renal biopsy cohort | |
|---|---|
| Diagnosis | Log AREG-to-creatinine ratio (pg/mgCr) |
| Minor glomerular change | 1.90 ± 1.03 |
| IgA nephropathy | 2.67 ± 1.38 |
| Diabetic nephropathy | 3.82 ± 1.08 |

### Example 4. Validation of prediction of progression to end-stage renal disease based on concentration of amphiregulin in urine of patient group who underwent renal biopsy

Using the urine samples at the time of biopsy of 72 patients diagnosed with minor glomerular change, IgA (immunoglobulin A) nephropathy, and diabetic nephropathy among the patients who underwent a renal biopsy, amphiregulin in urine was quantified through ELISA. Thereafter, prognosis of the patients was observed and classification was performed depending on the stage of chronic kidney disease. For classification of chronic kidney disease, reference was made to the KDIGO guideline, which is the representative guideline for the diagnosis and treatment of chronic kidney disease (Kidney Int. Suppl. 2012; 2013:1-150).

The urine amphiregulin concentration analyzed at the time of biopsy depending on the stage of chronic kidney disease was plotted, and the patients were divided into three groups (T1: 0.598-9.472 pg/mgCr, T2: 9.473-39.904 pg/mgCr, T3: 39.905-597.252 pg/mgCr) depending on the tertiles of urine amphiregulin concentration. Kaplan-Meier survival analysis was performed in order to determine whether there was a difference in the probability of progression to end-stage renal disease in the three groups. The ELISA analysis method was performed in the same manner as described in Example 2-1.

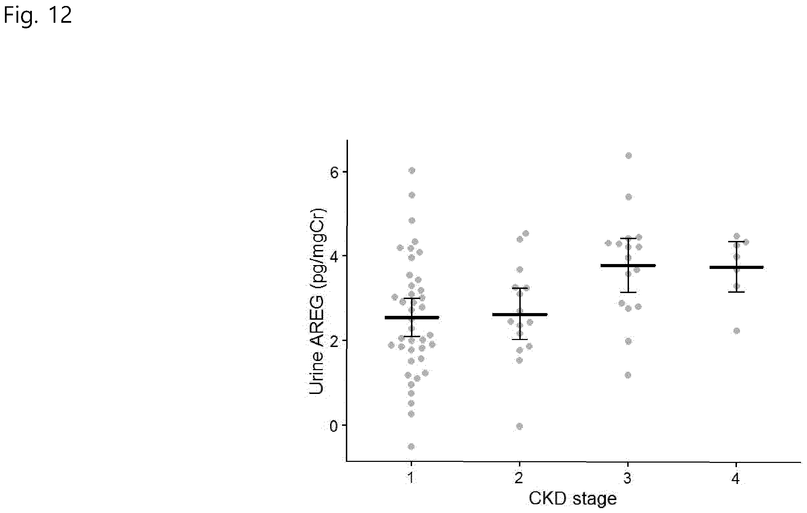

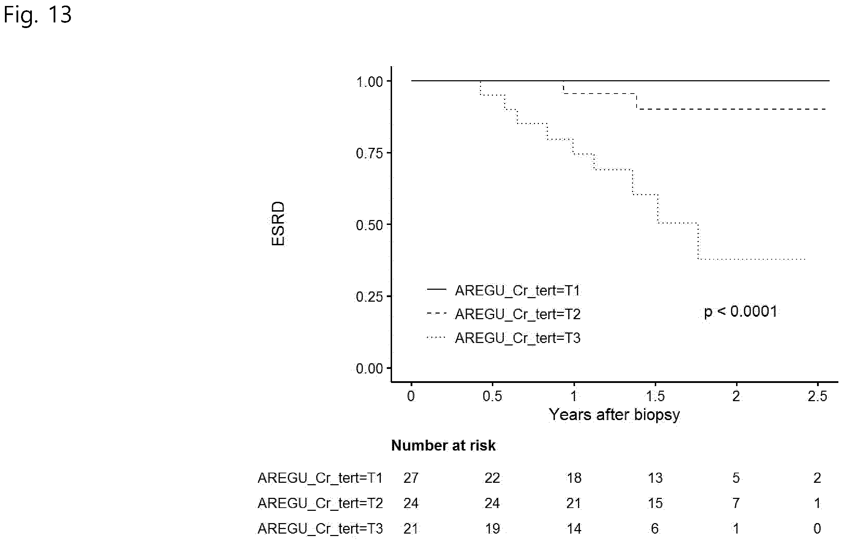

The results thereof are shown in FIG. 12, and chronic kidney disease of the patient group who underwent the renal biopsy was distributed in stages 1 to 4, and the concentration of amphiregulin in urine was measured to be higher in stage 3 and 4 patients with decreased kidney function than in stage 1 and 2 patients with relatively good kidney function. In addition, as is apparent from the Kaplan-Meier survival curve of FIG. 13, the incidence of end-stage renal disease was significantly high in the T3 group, which showed a high urine amphiregulin concentration among the tertiles of the urine amphiregulin concentration at the time of biopsy (ESRD incidence per 1000 person-years: T1, 0.0 [95% CI, 0.0-117.3]; T2, 71.4 [95% CI, 14.2-229.0]; T3, 600.0 [95% CI, 296.3-1095.1]).

Also, in order to improve the accuracy of the experiment, based on the results of correction for age, gender, urine baseline 24hr protein (creatinine), and baseline GFR, the progression rate of end-stage renal disease was significantly high in the T3 group between the T1/T2 integrated group and the T3 group among the tertile groups (hazard ratio between T3 and T1/T2 groups: 11.7 [95% CI 1.6-86.5]). In addition, when the log urine amphiregulin-to-creatinine ratio was set as a continuous variable and analyzed, it was found that there was a significant association with the incidence of chronic kidney disease even after correction of the above variables (hazard ratio whenever the log urine amphiregulin-to-creatinine ratio was increased by 1: 5.1 [95% CI 1.8-14.8]). When the amount of amphiregulin in the urine was 39.905 pg/mgCr or more in the patient group participating in this experiment, the probability of progression to end-stage renal disease after 2 years was determined to be 62.2% (FIG. 13).

Although specific embodiments of the present invention have been disclosed in detail above, it will be obvious to those skilled in the art that the description is merely of preferable exemplary embodiments and is not to be construed as limiting the scope of the present invention. Therefore, the substantial scope of the present invention is to be defined by the appended claims and equivalents thereof.

### [Industrial Applicability]

According to the present invention, kidney disease can be easily diagnosed in a non-invasive manner using a urine sample for kidney disease, early diagnosis of which is important. In addition, when the present invention is applied to a patient diagnosed with chronic kidney disease, the likelihood of progression to end-stage renal disease can be predicted in advance.

### [Sequence List Free Text]

An electronic file is attached.

## Claims

1. A composition for diagnosing kidney disease, comprising an agent that specifically detects amphiregulin or mRNA thereof.

2. The composition according to claim 1, wherein the agent specifically detects amphiregulin from a urine sample.

3. The composition according to claim 1, wherein the amphiregulin is represented by an amino acid sequence of SEQ ID NO: 1.

4. The composition according to claim 1, wherein the agent that specifically detects amphiregulin is an amphiregulin-specific antibody or aptamer.

5. The composition according to claim 1, wherein the kidney disease is selected from the group consisting of renal fibrosis, nephrotic syndrome, kidney cancer, chronic kidney disease, diabetic nephropathy, acute pyelonephritis, acute renal failure, end-stage renal disease, hypertensive kidney disease, Reye's syndrome, gout, Sjogren's syndrome, Behcet's disease, lupus, candidiasis, hemorrhagic fever with renal syndrome, leptospirosis, legionellosis, autosomal dominant polycystic kidney disease, and hydronephrosis.

6. A kit for diagnosing kidney disease, comprising the composition according to any one of claims 1 to 4.

7. The kit according to claim 6, wherein the kit is a protein chip kit or an immunodiagnostic kit.

8. The kit according to claim 6, wherein the kidney disease is selected from the group consisting of renal fibrosis, nephrotic syndrome, kidney cancer, chronic kidney disease, diabetic nephropathy, acute pyelonephritis, acute renal failure, end-stage renal disease, hypertensive kidney disease, Reye's syndrome, gout, Sjogren's syndrome, Behcet's disease, lupus, candidiasis, hemorrhagic fever with renal syndrome, leptospirosis, legionellosis, autosomal dominant polycystic kidney disease, and hydronephrosis.

9. A method of providing information for diagnosing kidney disease, comprising:
(a) taking a urine sample from a subject;
(b) detecting amphiregulin in the urine sample; and
(c) determining that the subject has kidney disease when an amount of amphiregulin that is detected in the urine sample is 5 pg/mgCr or more.

10. The method according to claim 9, wherein the detecting the amphiregulin is performed through an amphiregulin-specific reaction using an amphiregulin-specific antibody or aptamer.

11. The method according to claim 9, wherein the kidney disease is selected from the group consisting of renal fibrosis, nephrotic syndrome, kidney cancer, chronic kidney disease, diabetic nephropathy, acute pyelonephritis, acute renal failure, end-stage renal disease, hypertensive kidney disease, Reye's syndrome, gout, Sjogren's syndrome, Behcet's disease, lupus, candidiasis, hemorrhagic fever with renal syndrome, leptospirosis, legionellosis, autosomal dominant polycystic kidney disease, and hydronephrosis.

12. A method of providing information for predicting likelihood of progression to end-stage renal disease, comprising:
(a) taking a urine sample from a patient suffering from kidney disease;
(b) measuring an amount of amphiregulin in the urine sample; and
(c) determining that the patient is likely to progress to end-stage renal disease when the amount of amphiregulin that is measured is 30 pg/mgCr or more.
